# FASCICULE DE BREVET EUROPEEN

(11) **EP 0 466 583 B1**
(45) Date de publication et mention de la délivrance du brevet: **14.12.1994**
(21) Numéro de dépôt: 91401911.2
(22) Date de dépôt: 09.07.1991
(51) Int. Cl.: C07D 307/60

(54) **Procédé de préparation de la diméthyl-2,5 hydroxy-4 (2H)furanone-3**
Verfahren zur Herstellung von 2,5-Dimethyl-4-hydroxy-3(2H)-furanon
Process for the preparation of 2,5-dimethyl-4-hydroxy-3(2H)-furanone

(30) Priorité: 10.07.1990 FR 9008772
(43) Date de publication de la demande: 15.01.1992
(73) Titulaire: SANOFI, 75008 Paris (FR)
(72) Inventeur: Fellous, Roland, F-06800 Cagnes Sur Mer (FR); George, Gérard, F-06000 Nice (FR)
(74) Mandataire: Gillard, Marie-Louise

(56) Documents cités:
- FR-A- 2 211 457
- CHEMISTRY LETTERS, 1976, pages 495-498, Chemical Society of Japan, Tokyo, JP; S. TORII et al.: "A convenient preparation of maltol, ethylmaltol, and pyromeconic acid from 2-alkyl-6-methoxy-2H-pyran-3(6H)-ones"
- Tetrahedron, vol. 36 (1980) p. 1671-1680

## Description

La présente invention concerne un nouveau procédé de préparation de la diméthyl-2,5 hydroxy-4 (2H)furanone-3, composé à l'odeur de caramel, utilisé dans l'industrie alimentaire et l'industrie des parfums.

Divers procédés de préparation de ce produit ont été proposés; ainsi, dans Helv. Chim. Acta 49 p. 53 - 56(1966), on décrit sa préparation à partir de l'hexanetrione-2,3,5 ol-4.

Dans les brevets suisses CH 474 500 et 474 501, on décrit son obtention par cyclisation des hexanedioldiones : CH₃COCHOHCHOHCOCH₃ et CH₃CHOHCOCOCHOHCH₃ et, dans les brevets allemands DE 2 105 014 et 2 831 673, par cyclisation des dérivés de formule CH₃CHXCOCOCHXCH₃, dans laquelle X représente Cl ou Br. On a aussi proposé sa préparation par oxydation des dihydrofuranones de formule A : 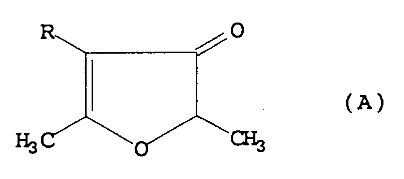
dans laquelle R = CN, dans le brevet CA 1 132 591 ou R = COOH dans le brevet FR-A-2 211 457.

Le procédé selon l'invention met aussi en jeu l'oxydation d'une dihydrofuranone, mais le procédé de préparation de cette dernière est plus simple que celle des composés de formule A.

On a par ailleurs décrit dans Tetrahedron, vol. 36, 1980, pages 1671-1680 l'oxydation par l'eau oxygénée en milieu basique de la méthyl-2 chromone qui conduit à la méthyl-2 hydroxy-3 chromone.

Le procédé de l'invention consiste à faire réagir sur la diméthyl-2,5 (2H)furanone-3 de formule I : 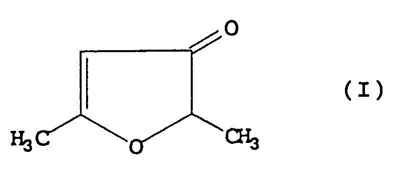
du peroxyde d'hydrogéne, en présence d'une base.

La réaction est avantageusement effectuée en phase homogène, à une température comprise entre 20°C et 80°C environ. Comme solvant, on peut utiliser un mélange d'eau et d'alcool, tel que le méthanol ou l'éthanol ou d'eau et de cétone, telle que l'acétone. Parmi les bases utilisables, on peut citer les hydroxydes et les carbonates alcalins.

La diméthyl-2,5 (2H)furanone-3 est un produit connu, qui peut être préparé par exemple, à partir du diacétyle H₃CCOCOCH₃, selon le procédé décrit dans Synthesis p. 754 (1977) ou par action d'un halogénure de l'acide bromo-2 propanoïque sur le sel de sodium de l'acide oxo-3 butanoique, selon le procédé décrit dans le brevet FR-A-2 211 457 ou encore à partir d'un ester de l'acide diméthyl-2,5 furanne-3 carboxylique comme mentionné dans Helv. Chim. Acta 46 1259 (1963).

Dans ce qui suit on décrit des exemples de mise en oeuvre de l'invention.

### Exemple 1

Dans une solution de 304 g de bicarbonate de potassium dans 420 ml de méthanol et 420 ml d'eau, on introduit 378 g de diméthyl-2,5 (2H)furanone-3 puis en 1 heure, sous agitation, en maintenant la température entre 30° et 40°C, 372 ml d'eau oxygénée à 30 % (p/p).

Après 2 heures d'agitation, vers 20°C, on extrait, dans un appareil d'extraction liquide-liquide, le produit de départ et le produit cherché du milieu réactionnel dans 500 ml d'acétate d'éthyle.

Le solvant est éliminé sous vide pour donner 160 g d'une huile brunâtre ; celle-ci est distillée sous pression réduite. La fraction distillant vers 100°C sous 250 Pa est isolée et mise à cristalliser dans deux fois son poids d'acétate d'éthyle ou d'éther isopropylique.

On obtient ainsi 25 g de diméthyl-2,5 hydroxy-4 (2H)-furanone-3.

Dans la fraction ayant distillé avant 100°C, on récupère 40 g de produit de départ qui peut être recyclé.

### Exemple 2

On dissout 56 g de diméthyl-2,5 (2H)furanone-3 dans 390 ml de méthanol et on mélange cette solution avec 110 ml d'eau oxygénée à 30 % (p/p). Sous agitation, on introduit dans ce milieu une solution aqueuse d'hydroxyde de sodium (9,4 g NaOH en pastilles dans 33 g d'eau). Après 4 heures 30 d'agitation, à 60°C, le rendement en produit final est de 12 %.

### Exemple 3

On opère comme à l'exemple 2, mais en utilisant l'éthanol au lieu du méthanol comme solvant ; le rendement est alors de 4 %.

### Exemple 4

On introduit 56 g de diméthyl-2,5 (2H)furanone-3 dans un mélange de 390 ml de méthanol et 140 g d'une solution saturée de carbonate de sodium, puis, en 1 heure, 111 ml d'eau oxygénée à 30 % (p/p) ; après 4 heures, à 70°C, on constate par chromatographie en phase gazeuse que le rendement en produit attendu est de 11 %.

## Revendications

1. Procédé de préparation de la diméthyl-2,5 hydroxy-4 (2H)furanone-3, caractérisé en ce que l'on fait réagir du peroxyde d'hydrogène sur la diméthyl-2,5 (2H)furanone-3 en présence d'une base.

2. Procédé selon la revendication 1, caractérisé en ce que le milieu réactionnel est homogène.

3. Procédé selon l'une des revendications 1 ou 2, caractérisé en ce que la base est un hydroxyde ou un carbonate alcalin, et que le solvant est un mélange d'alcool et d'eau ou de cétone et d'eau.

4. Procédé selon la revendication 1, caractérisé en ce que la base est l'hydroxyde de sodium et en ce que la réaction est effectuée en phase homogène dans un mélange de méthanol et d'eau.

## Claims

1. Method of preparing 2,5-dimethyl-4-hydroxy-2H-furan-3-one, characterized in that hydrogen peroxide is reacted with 2,5-dimethyl-2H-furan-3-one in the presence of a base.

2. Method according to claim 1, characterized in that the reaction medium is homogeneous.

3. Method according to one of claims 1 or 2, characterized in that the base is an alkaline hydroxide or carbonate and in that the solvent is a mixture of an alcohol and water or of a ketone and water.

4. Method according to claim 1, characterized in that the base is sodium hydroxide and in that the reaction is carried out in a homogeneous phase in a mixture of methanol and water.

## Patentansprüche

1. Verfahren zur Herstellung von 2,5-Dimethyl-4-hydroxy-(2H)-furan-3-on, gekennzeichnet durch Umsetzung von 2,5-Dimethyl-(2H)-furan-3-on mit Wasserstoffperoxid in Gegenwart einer Base.

2. Verfahren nach Anspruch 1, dadurch gekennzeichnet, daß das Reaktionsmilieu homogen ist.

3. Verfahren nach Anspruch 1 oder 2, dadurch gekennzeichnet, daß die Base ein Alkalihydroxid oder ein Alkalicarbonat ist und das Lösungsmittel ein Alkohol-Wasser-Gemisch oder ein Keton-Wasser-Gemisch darstellt.

4. Verfahren nach Anspruch 1, dadurch gekennzeichnet, daß die Base Natriumhydroxid ist und die Umsetzung in homogener Phase in einem Methanol-Wasser-Gemisch vorgenommen wird.
